# EUROPÄISCHE PATENTSCHRIFT

(11) **EP 3 496 686 B1**
(45) Veröffentlichungstag und Bekanntmachung des Hinweises auf die Patenterteilung: **15.07.2020**
(21) Anmeldenummer: 17749709.6
(22) Anmeldetag: 09.08.2017
(51) Int. Cl.: A61F 13/00, A61M 1/00

(54) **SAUGKÖRPER ZUR ENDOLUMINALEN UNTERDRUCKTHERAPIE**
SUCTION ELEMENT FOR ENDOLUMINAL NEGATIVE PRESSURE THERAPY
CORPS D'ASPIRATION DESTINÉ À UN TRAITEMENT PAR PRESSION NÉGATIVE ENDOLUMINAL

(30) Priorität: 10.08.2016 DE 102016114819
(43) Veröffentlichungstag der Anmeldung: 19.06.2019
(73) Patentinhaber: Paul Hartmann AG, 89522 Heidenheim (DE)
(72) Erfinder: GÖRL, Rudolf, 67853 Obernburg (DE); ECKSTEIN, Axel, 89522 Heidenheim (DE)
(74) Vertreter: Eibl, Christian
(86) Internationale Anmeldenummer: PCT/EP2017/070139
(87) Internationale Veröffentlichungsnummer: WO 2018/029231

(56) Entgegenhaltungen:
- EP-A1- 2 769 744
- WO-A1-2010/063466
- WO-A1-2011/038949
- WO-A1-2013/181686
- RUDOLF MENNIGEN ET AL: "Novel treatment options for perforations of the upper gastrointestinal tract: Endoscopic vacuum therapy and over-the-scope clips", WORLD JOURNAL OF GASTROENTEROLOGY, Bd. 20, Nr. 24, 28. Juni 2014 (2014-06-28) , Seiten 7767-7776, XP055410851, CN ISSN: 1007-9327, DOI: 10.3748/wjg.v20.i24.7767 in der Anmeldung erwähnt

## Beschreibung

Die Erfindung betrifft eine Vorrichtung zur Verwendung bei der Unterdrucktherapie von endoluminalen Wundstellen im Gastrointestinaltrakt.

Unterdrucktherapie von Wunden, im Fachbereich auch bezeichnet als topische negative Druckbehandlung (TNP = topical negative pressure) oder NPWT (negative pressure wound therapy), ist seit langem bekannt, findet aber insbesondere seit Mitte/Ende der 1990er Jahre in zunehmendem Maße Anwendung. Dabei wird üblicherweise ein Wundfüllmaterial in die Wunde eingelegt, das Wundgebiet mit einer Folie abgedeckt und mittels eines Drainageschlauchs und einer Vakuumpumpe ein Unterdruck im Wundraum erzeugt. Durch die Verwendung des Wundfüllmaterials wird der Druck gleichmäßig über die Wunde verteilt. Der Unterdruck bewirkt, insbesondere in der initialen Phase der Wundbehandlung, eine effektive Wundreinigung durch Abtransport von Wundexsudat. Als weitere Vorteile werden, insbesondere in der nachfolgenden Granulationsphase, Förderung der Bildung von Granulationsgewebe und Verminderung der Wundödembildung genannt.

Unterdrucktherapie kann bei akuten oder bei chronischen Wunden eingesetzt werden. Hierbei können auch infizierte Wunden erfolgreich behandelt werden. Insbesondere wird die Unterdrucktherapie eingesetzt bei Geschwüren (Ulcus, z.B. Druckgeschwüren, diabetischen Geschwüren, neuropathischen Geschwüren oder venös bedingten Geschwüren), Druckwunden (Dekubitus), traumatischen Wunden, therapieresistenten Wunden, postoperativen Wunden, sondierten Fisteln sowie Hautlappen und Hauttransplantaten. Chronische Wunden sind durch eine verlangsamte oder fehlende Wundheilung gekennzeichnet.
Vorrichtungen zur Unterdrucktherapie von Wunden sind entsprechend im Stand der Technik bekannt. Geeignete Unterdrucktherapiegeräte und Zubehör werden beispielsweise in den Dokumenten WO2011018132, WO2011018133, WO2012156140, WO2012156174, WO2012163617 und WO2013159904 beschrieben. In den Wundraum können beispielsweise die aus den Dokumenten WO2010072309, WO2012022484, WO2011072840, WO2012167942 oder WO2011003521 bekannten Wundauflagen eingebracht werden. Als besonders vorteilhaft in der Unterdruck-Wundbehandlung hat sich nach der Erfahrung des Anmelders eine Wundauflage aus einem offenzelligen Polyester-Polyurethanschaum, die in der EP2515812B1 (aus WO2012022485) näher beschrieben ist, erwiesen.
Geräte zur Unterdrucktherapie von Wunden sind auch kommerziell erhältlich und umfassen kleine, tragbare Geräte, die den Patienten eine gewisse Mobilität ermöglichen bis hin zu stationär zu verwendenden Geräten, etwa in Langzeitpflegeeinrichtungen. Auch für die Unterdrucktherapie geeignetes Zubehör wie beispielsweise Wundauflagen, weitere Verbandkomponenten oder Anschlussmittel sind kommerziell erhältlich.

Ein neues Anwendungsgebiet eröffnet sich für die Unterdrucktherapie bei der Behandlung von Wundstellen im Lumen des Verdauungstraktes. Indikationen für eine Unterdruckapplikation im Gastrointestinaltrakt umfassen Entzündungen, Verätzungen, Anastomose-Insuffizienzstellen und Tumorexzissionsstellen. Chirurgische Anastomosen entstehen nach Resektion von Abschnitten des Verdauungstrakts. Eine gastrointestinale Anastomoseinsuffizienz, also eine mangelnde Dichtheit der chirurgisch geschaffenen Verbindung zwischen Abschnitten des Verdauungstraktes, kann als postoperative Komplikation beispielsweise auf eine entzündungs- oder ischämisch bedingte Nahtinsuffizienz zurückgehen. Bei einem Austritt des Inhalts von Magen oder Darm in eine Körperhöhle drohen lebensgefährliche Entzündungen, beispielsweise eine lebensgefährliche Bauchfellentzündung (Peritonitis) bei einem Austritt von Darminhalt in die Bauchhöhle. Weitere mögliche Komplikationen umfassen Abszesse und schlimmstenfalls eine Sepsis. Zu den bisherigen Behandlungsansätzen beim Auftreten von Anastomoseinsuffizienzen gehören beispielsweise eine weitere chirurgische Intervention, die Platzierung eines Stents oder eine medikamentöse Behandlung. Die Erfolgsaussichten der genannten Therapien sind unbefriedigend.
Deutlich verbesserte Heilungschancen bietet inzwischen eine Behandlungsoption zur Versorgung von Anastomoseinsuffizienzen des Gastrointestinaltraktes, welche auf einer endoluminal gesetzten Schaumdrainage bei gleichzeitiger Applikation von Unterdruck beruht. Diese Behandlungsmethode wird auch als endoluminale Vakuumtherapie (EVT) oder als endoluminale Unterdrucktherapie bezeichnet. Eine Übersicht zu neuartigen Behandlungsverfahren bei Perforationen des oberen Gastrointestinaltraktes findet sich beispielsweise in Menningen R, Senninger N und Laukötter MG (2014) World Journal of Gastroenterology, Vol. 20, No. 24, S. 7767 - 76. Fallberichte zum erfolgreichen Einsatz der endoluminalen Vakuumtherapie bei ösophagaler Perforation werden beispielsweise in Hampe J (2014) Annals of Thoracic Surgery, Vol. 97, No. 3, S. 1029 -1035 beschrieben.

Anastomoseinsuffizienzen des Gastrointestinaltraktes umfassen kleinere Undichtigkeiten an der Nahtstelle bis hin zu einer ausgeprägten Abzesshöhle. Im Falle von Kavitäten, welche seitlich aus dem Lumen der Verdauungsorgane abzweigen können, wurden Volumina von 40 cm³ bis hin zu 160 cm³ beschrieben, wobei sich die Aussackung auf eine Länge von bis zu 8 cm erstrecken kann. Die Öffnungen der Kavität weisen typischerweise Durchmesser im Bereich von 2 - 4 cm auf.
Bei einer beabsichtigten Behandlung einer endoluminalen Wundstelle mittels Unterdruck muss ein mit dem stromaufwärts gelegenen Ende eines Absaugschlauches verbundener Fluidsammelkörper geeigneter Größe bereitgestellt und passgenau zur Zielstelle gebracht werden.

Die Ausdrücke "stromaufwärts" und "stromabwärts" sind im Zusammenhang mit der vorliegenden Anmeldung auf den Fluidstrom bezogen, welcher bei der hier beschriebenen Unterdrucktherapie endoluminaler Wundstellen ausgehend von der Wundstelle bzw. Wundkavität aus dem Körper heraus und weiter zur Unterdruckpumpe hin bewegt wird. Das stromaufwärts gelegene Ende des Absaugschlauches befindet sich demnach während der Unterdrucktherapie an der Wundstelle oder nahe der Wundstelle. Das stromabwärts gelegene Ende des Absaugschlauches ragt bei der Anwendung der Vorrichtung normalerweise aus dem Körper des Patienten heraus.

Ein mit einem Absaugschlauch verbundener Fluidsammelkörper wird im Folgenden auch als "Saugkörper" bezeichnet.
Als Fluidsammelkörper wird üblicherweise ein Schwammkörper, welcher insbesondere einen porösen Polymerschaumstoff umfasst, verwendet. Bei der Zielstelle kann es sich im einfacheren Falle um das Innere des Hauptlumens eines Abschnittes des Gastrointestinaltraktes handeln, beispielsweise um die Speiseröhre, um den Dickdarm oder um den Dünndarm.

Eine besondere Herausforderung für den behandelnden Arzt ergibt sich bei der Applikation des Saugkörpers in eine aus dem Hauptlumen abzweigende Aussackung. Eine derartige Aussackung kann insbesondere infolge einer Anastomoseinsuffizienz entstehen. Der Saugkörper kann nur dann in die Kavität eingeführt werden, wenn die Aussackung spiegelbar, d.h. mit einem Endoskop zugänglich ist. Die Größe des Fluidsammelkörpers muss an die Abmessung der Kavität angepasst sein. Die Abmessung der sackartig beschaffenen Kavität wird vor der Applikation des Saugkörpers endoskopisch ermittelt. Nach dem Einführen des Saugkörpers in das zu behandelnde, von der Speiseröhre oder von dem Darm abzweigende Lumen wird das aus dem Patienten herausragende Ende des Absaugschlauches mit einer Unterdruckquelle verbunden und Unterdruck angelegt. Infolge des Unterdrucks kollabiert die zu behandelnde Kavität teilweise, wobei sich ein enger Kontakt zwischen der Wand der Kavität und dem Fluidsammelkörper einstellt. Der Kontakt zwischen Fluidsammelkörper und der Wand des Lumens stimuliert die Bildung von Granulationsgewebe. Wundfluide werden über den Fluidsammelkörper und den Saugschlauch abgesaugt, so dass die Wunde permanent gesäubert wird und Infekte unter Kontrolle gehalten werden. Des Weiteren führt die Unterdruckapplikation zu einer kontinuierlichen Konstriktion der Ausstülpung. Der Saugkörper muss nach einigen Tagen kontinuierlicher Unterdruckapplikation immer wieder ersetzt werden, wobei die Größe des Fluidsammelkörpers bei fortschreitender Verkleinerung der Kavität schrittweise reduziert wird. Die Unterdruckbehandlung kann beendet werden, wenn die Aussackung weitestgehend zurückgegangen ist.

Im Falle einer Behandlung einer Anastomoseinsuffizienz ohne Aussackung wird die Therapie beendet, wenn die Leckage geschlossen ist.

Die Erfolgschancen des endoskopischen Eingriffs hängen wesentlich von der Auswahl eines passend dimensionierten Fluidsammelkörpers sowie von einer optimalen Platzierung des Saugkörpers in der Wundhöhle ab. Um den Saugkörper in die Kavität einzuführen, sind aus der medizinischen Praxis unterschiedliche Ansätze bekannt.
Die EP1572286-B1 beschreibt ein Behandlungssystem zur endoskopischen Wundversorgung, welches insbesondere zur Behandlung perianastomotischer Abszesse vorgesehen ist. Das Behandlungssystem umfasst einen Fluidsammelkörper, einen mit dem Fluidsammelkörper verbundenen Absaugschlauch sowie eine Einführhilfe (Inserter). Die Einführhilfe besteht aus einem dickeren Schlauch (Outer Sleeve bzw. Overtube) und aus einem dünneren Schlauch (Inner Sleeve bzw. Pusher). Der Overtube wird über ein bewegliches Endoskop in die Wundhöhle geschoben. Nach dem Entfernen des Endoskops wird der Fluidsammelkörper durch den Overtube geschoben und mithilfe des Pusher positioniert bzw. freigesetzt. Ein derartiges endoskopisches Behandlungssystem ist unter der Bezeichnung Endo-SPONGE® für den unteren Gastrointestinaltrakt und unter der Bezeichnung Eso-SPONGE® für den oberen Gastrointestinaltrakt im Handel erhältlich (Firma B. Braun Melsungen AG, Deutschland). Ein Einführsystem mit Positionierungshülse und Führungshülse ist beispielsweise auch aus der DE102009043472 bekannt.
Ein alternatives Verfahren zur präzisen Platzierung des Saugkörpers an der gewünschten Wundstelle beruht darauf, den am Saugkörper vorhandenen Schwamm mit einer am Ende eines endoskopischen Instrumentes angebrachten Zange zu ergreifen und dann mittels des Endoskops in die Wundtasche zu ziehen. Gemäß einer in der Praxis üblichen Vorgehensweise wird hierbei unmittelbar vor der endoskopischen Behandlung durch den behandelnden Arzt unter Einsatz chirurgischen Nahtmaterials am Wundschwamm eine Fadenschlaufe angebracht. Der Faden wird hierbei mit dem Schwamm und normalerweise auch mit dem Absaugschlauch vernäht, um ein Ausreißen des Schwamms zu vermeiden. Die EP2769744 beschreibt ein Medizinprodukt zur Drainage pathologischer Flüssigkeitsansammlungen, welches einen Fluidsammelkörper und einen mit dem Fluidsammelkörper verbundenen Absaugschlauch umfasst. Innerhalb des Absaugschlauches ist ein Faden vorhanden, welcher schlaufenförmig aus dem bei der Behandlung zur Wundhöhle hin zeigenden Ende des Absaugschlauches heraushängt. Die Fadenschlaufe dient als Fassschlaufe zur Positionierung des Saugkörpers mittels eines Endoskops.

Die WO2010063466 beschreibt einen für die endoluminale Vakuumtherapie geeigneten absorbierenden Schwammkörper, welcher mittels einer Hülle in einem komprimierten Zustand gehalten wird. Der Schwammkörper wird in dem komprimierten Zustand an seine Zielstelle in der zu behandelnden Kavität verbracht. Nach dem Ablösen seiner Hülle kann sich der Schwammkörper ausdehnen und die Kavität ausfüllen. Die Fixierung des Schwammkörpers an dem Absaugschlauch erfolgt durch Vernähen oder Verkleben.

Die WO2011038949 offenbart eine für die endoluminale Vakuumtherapie vorgesehene Vorrichtung, welche einen Schwammkörper und einen wenigstens teilweise in dem Schwammkörper vorhandenen Absaugschlauch mit Perforationen umfasst. Der Schwammkörper weist ferner einen Kanal zum Führen eines Führungselementes oder eines Versorgungselementes auf. Der Schwammkörper ist mit dem Drainageschlauch fluidleitend und vorzugsweise fest verbunden, insbesondere durch Verkleben, Verschweißen oder Vernähen.

Bezüglich der Größenanpassung ist es derzeit in der Praxis üblich, einen kommerziell erhältlichen Schwammkörper vor der Anwendung mittels einer Schere oder mittels eines Skalpells an die endoskopisch ermittelten Abmessungen (Durchmesser und/oder Länge des Schwammkörpers) des zu behandelnden Wundtraumes anzupassen.

Der vorliegenden Erfindung lag die Aufgabe zugrunde, eine Vorrichtung bereit zu stellen, um die endoluminale Unterdrucktherapie weiter zu verbessern. Insbesondere sollte die Handhabung der endoskopischen Komponenten vereinfacht werden, die Dauer des endoskopischen Eingriffes verkürzt und die Anwendungssicherheit erhöht werden.

Die Erfindung löst die oben genannten Aufgaben durch Bereitstellung einer Vorrichtung zur Verwendung bei der Unterdrucktherapie von endoluminalen Wundstellen im Bereich des Gastrointestinaltraktes gemäß Anspruch 1.
Die erfindungsgemäße Vorrichtung umfasst einen Schwammkörper, welcher zum Auskleiden einer Wundhöhle und zur Aufnahme von Fluiden aus dieser Wundhöhle vorgesehen ist. Bei den aus der Wundhöhle aufzunehmenden Fluiden handelt es sich insbesondere um flüssige und zähflüssige Substanzen, beispielsweise um Blut, Schleim, Eiter, aber auch um von außen während der Behandlung zugeführte Flüssigkeiten. Bei dem Schwammkörper handelt es sich also um einen Fluidsammelkörper. Der Schwammkörper umfasst vorzugsweise einen polymeren Schaumstoff, insbesondere einen offenzelligen Polymerschaum aus Polyurethan oder Polyvinylalkohol. Der Schwammkörper kann vorzugsweise auch vollständig aus einem polymeren Schaumstoff bestehen. Besonders bevorzugt wird ein Polymerschaumstoff aus einem Polyester-Polyurethan eingesetzt, wie beispielsweise in der EP2515812B1 beschrieben.
Weiterhin umfasst die erfindungsgemäße Vorrichtung herstellerseitig einen mit dem Schwammkörper unlösbar verbundenen Absaugschlauch. Der Absaugschlauch dient zur Beaufschlagung der Wundstelle mit Unterdruck und zum Abtransport der von dem Fluidsammelkörper aufgenommenen Substanzen. Hierzu weist der Absaugschlauch eine oder mehrere Öffnungen auf.
Der Absaugschlauch weist entlang seiner Achse mindestens einen ersten, einen zweiten und einen dritten Abschnitt auf.
Herstellerseitig umschließt der Schwammkörper den ersten und den zweiten Abschnitt des Absaugschlauches. Der erste und der zweite Abschnitt des Absaugschlauchs verlaufen also innerhalb des Schwammkörpers. Der Absaugschlauch umfasst einen dritten Abschnitt, welcher nicht vom Schwammkörper umschlossen ist. Der dritte Abschnitt ist stromabwärts des ersten und des zweiten Abschnitts angeordnet. Der dritte Abschnitt des Absaugschlauchs wird aus dem Gastrointestinaltrakt des Patienten herausgeleitet.

Zumindest eine im Absaugschlauch vorhandene Öffnung befindet sich im Bereich des ersten Abschnittes des Absaugschlauchs. Vorzugsweise ist im Bereich des ersten Abschnittes des Absaugschlauchs mehr als eine Öffnung vorhanden, insbesondere sind im Bereich des ersten Abschnittes des Absaugschlauchs 2 bis 20 Öffnungen vorhanden. Da die mindestens eine im Bereich des ersten Abschnittes vorhandene Öffnungen an einem innerhalb des Schwammkörpers verlaufenden Abschnitts des Absaugschlauchs lokalisiert ist, werden die Fluide durch den Schwammkörper hindurch zum Absaugschlauch gesaugt. Weitere Öffnungen können im Bereich des zweiten und/oder des dritten Abschnittes des Absaugschlauchs vorhanden sein. Zusätzlich kann der Absaugschlauch eine an seinem wundseitigen Ende vorhandene endständige Öffnung aufweisen. Der Absaugschlauch fungiert somit als medizinischer Drainageschlauch.
Der Schwammkörper ist herstellerseitig mit dem Absaugschlauch mittels Klebstoffauftrag unlösbar verbunden.
Erfindungsgemäß ist die Vorrichtung dadurch gekennzeichnet,
dass der Absaugschlauch im Bereich des ersten Abschnitts herstellerseitig mittels Klebstoffauftrag mit dem Schwammkörper unlösbar verbunden ist,
und weiter dadurch, dass der Absaugschlauch im Bereich des zweiten Abschnitts keinen Klebstoffauftrag aufweist. Hierdurch kann der Schwammkörper durch den Anwender im Bereich des zweiten Abschnitts vom Ende her bedarfsgemäß eingekürzt werden, um die Länge des Schwammkörpers an die zu behandelnde Wundstelle anzupassen. Es ist auch möglich, dass der Anwender den über dem gesamten zweiten Abschnitt des Absaugschlauchs vorhandenen Schwamm entfernt.

Im Zusammenhang mit der Erfindung können für den Klebstoffauftrag übliche Klebstoffe verwendet werden, welche für medizinische Anwendungen geeignet sind und welche eine stabile Verbindung zwischen Schwammkörper und Absaugschlauch (auch nach einer Sterilisation der Vorrichtung) gewährleisten.

Erfindungsgemäß ist der erste Abschnitt des Absaugschlauchs im Fluidsystem stromaufwärts des zweiten Abschnitts vorhanden. Der mit einem Klebstoffauftrag versehene erste Abschnitt des Absaugschlauchs ist also im Vergleich zu dem zweiten Abschnitt des Absaugschlauchs näher an der Wundstelle. Die Längenanpassung des Schwammkörpers erfolgt vom stromabwärts vorhandenen Ende her.

Die Längenanpassung des Schwammkörpers erfolgt bei der erfindungsgemäß vorgeschlagenen Vorrichtung unmittelbar vor Gebrauch der Vorrichtung zur Unterdrucktherapie und kann beispielsweise mittels einer Schere oder mittels eines Skalpells durchgeführt werden. Die Längenanpassung kann durch den Anwender, üblicherweise ein Arzt, schnell und einfach durchgeführt werden. Die Vorbereitungszeit für den Eingriff kann verkürzt werden. Der Schwammkörper bleibt während der Unterdruckbehandlung mit dem ersten Abschnitt des Absaugschlauches fest verbunden.
Die erfindungsgemäße Lösung überwindet zudem aus dem Stand der Technik bekannte Probleme bei der Längenanpassung des Schwammkörpers: Bei einer Längenanpassung des Schwammkörpers vom stromabwärts vorhandenen Ende her konnten bei den aus dem Stand der Technik bekannten Schwammkörpern mit dem Absaugschlauch mittels Klebstoffauftrag verbundene Materialanteile (insbesondere Schaumstoff) nicht vollständig entfernt werden. Bei einer Längenanpassung des Schwammkörpers vom stromaufwärts vorhandenen Ende her wurde die Struktur des wundnahen Endbereichs des Schwammkörpers in unerwünschter Weise verändert. So wurden durch das Zuschneiden des Schwammkörpers vom wundnahen Ende her beispielsweise am Ende vorhandene Greiffmittel abgeschnitten. Ein gegebenenfalls aus dem Schwammkörper herausragender Anteil des Absaugschlauches wurde bei dem aus der Stand der Technik bekannten Schwammkörpern abgeschnitten. Ein gegebenenfalls im Schwammkörperende endender Absaugschlauch wurde freigelegt.

Bei der erfindungsgemäßen Vorrichtung ist vorzugsweise an dem bei Gebrauch der Vorrichtung in der Unterdruckbehandlung aus dem Patienten herausragenden Ende des dritten Abschnitts des Absaugschlauchs ein Konnektor zum Verbinden des Absaugschlauches mit einer Unterdruckquelle vorhanden. Bei dem Konnektor handelt es sich insbesondere um einen Schnelltrennverbinder. Der Konnektor ist an demjenigen Ende des Absaugschlauches angebracht, welches bei Gebrauch der Vorrichtung außerhalb des Patienten vorhanden ist.

Vorzugsweise umfasst der erste Abschnitt des Saugschlauches 10 % bis 90 % der entlang der Achse des Absaugschlauches gemessenen Summe der Längen von erstem und zweitem Abschnitt des Absaugschlauchs. Besonders bevorzugt umfasst der erste Abschnitt des Saugschlauches 25 % bis 80 % der entlang der Achse des Absaugschlauches gemessenen Summe der Längen von erstem und zweitem Abschnitt des Absaugschlauchs, insbesondere 40 % bis 70 %. In der Praxis hat es sich als besonders vorteilhaft erwiesen, wenn der erste und der zweite Abschnitt des Absaugschlauches entlang der Achse des Absaugschlauches eine im Wesentlichen gleiche Länge aufweisen. Hierdurch ergibt sich eine vorteilhafte Kombination aus Stabilität der Klebestelle und einer ausreichenden Möglichkeit zur Längenanpassung des Schwammkörpers.

Der Absaugschlauch kann optional einen weiteren (vierten) Abschnitt umfassen, welcher stromaufwärts des ersten Abschnitts vorhanden ist und welcher von dem Schwammkörper nicht umschlossen ist. Dies bedeutet, dass bei dieser Variante der Erfindung ein Teilstück des Absaugschlauchs wundseitig aus dem Schwammkörper herausragt.

Gemäß einer besonders bevorzugten Ausführungsform der Erfindung ist die Vorrichtung weiter dadurch gekennzeichnet, dass an der Vorrichtung eine bereits herstellerseitig angebrachte Fassschlaufe vorhanden ist. Die Fassschlaufe ist hierbei entweder aus einem Abschnitt des Schwammkörpers, oder aus einem Abschnitt des Absaugschlauchs oder aus einem mit dem Absaugschlauch unlösbar verbundenem Materialabschnitt geformt. Im Zusammenhang mit der Erfindung hat sich als besonders vorteilhaft erwiesen, wenn die Fassschlaufe aus dem aus dem Schwammkörper herausragenden vierten Abschnitt des Absaugschlauch geformt wird, sofern der vierte Abschnitt des Absaugschlauches vorhanden ist. Die Fassschlaufe dient zum Ergreifen der Vorrichtung und dann zum Führen des Schwammkörpers zur Wundstelle hin, wobei das Ergreifen und das zum Zielort hin translozieren üblicherweise durch ein endoskopisches Instrument unter Kamerakontrolle erfolgt. Hierzu weist das endoskopische Instrument an seinem Ende eine miniaturisierte Greifzange auf, welche die Fassschlaufe fest, jedoch reversibel packen und zum Zielort mitziehen kann. Es geht also bei der vorliegenden Ausführungsform insbesondere um eine bereits herstellerseitig an der Vorrichtung zur endoluminalen Unterdrucktherapie vorhandene Fassschlaufe, die mit einem medizinischen Instrument ergriffen werden kann. Unter dem Begriff "Fassschlaufe" wird im Zusammenhang mit der vorliegenden Erfindung allgemein eine griffförmige Struktur verstanden, welche geeignet ist, von einem an einem endoskopischen Instrument vorhandenen Greifmechanismus, insbesondere von einer Greifzange, reversibel festgehalten zu werden. Die Fassschlaufe soll hinsichtlich ihrer Abmessungen und ihrer Form an die an dem endoskopischen Instrument vorhandene Greifzange angepasst werden.

Im Gegensatz zu bisher üblichen Verfahren, bei denen der behandelnde Arzt einen Faden an den Saugkörper angebracht hat, ist die nach dieser bevorzugten Ausführungsform vorgestellte Methode schneller, zuverlässiger und sicherer. Der Arzt kann sich vollständig auf die bevorstehende Operation konzentrieren und muss sich nicht mit der Vorbereitung des Wundkörpers befassen. Die Fassschlaufe ist stabil und kann optimal auf das endoskopische Instrument abgestimmt werden. Ein unbeabsichtigtes Abfallen des Schwammes von dem endoskopischen Instrument wird weitestgehend vermieden. Die Fassschlaufe ermöglicht eine präzise Applikation des Schwammkörpers mittels eines flexiblen Endoskops. Ebenso gewährleistet eine auf das endoskopische Instrument abgestimmt Fassschlaufe eine unproblematische Freisetzung des Saugkörpers am Zielort: Im Gegensatz zu herkömmlichen Methoden wird die Gefahr reduziert, dass sich Fadenschlingen an der Greifzange des endoskopischen Instrumentes verheddern und so das Abkoppeln des Saugkörpers vom Instrument beeinträchtigen.

Gemäß einer ersten Variante der vorgenannten bevorzugten Ausführungsform wird die Fassschlaufe herstellerseitig aus dem vierten Abschnitt des Absaugschlauchs geformt, welcher wundseitig aus dem Schwammkörper herausragt. Hierbei ist es besonders bevorzugt, dass die Fassschlaufe durch Stanzen einer Öffnung in den Absaugschlauch geformt wird. Die eingestanzte Öffnung kann in Längsrichtung des Absaugschlauches einen Durchmesser von 2 mm bis 200 mm aufweisen. Vorzugsweise beträgt ihre Länge zwischen 3 mm und 25 mm, wobei die Länge der Öffnung an die Abmessungen der Greifzange des endoskopischen Instrumentes abgestimmt ist. Der maximale Durchmesser der Stanzöffnung in Querrichtung hängt von der Dicke des Absaugschlauches ab. Der Durchmesser der eingebrachten Öffnung in Querrichtung darf den Durchmesser des Lumens des Absaugschlauches nicht überschreiten, um die Stabilität der Fassschlaufe zu gewährleisten. So darf beispielsweise bei einem Absaugschlauch mit einem äußeren Durchmesser von 5 mm und einem inneren Durchmesser des Lumens von 3 mm der Durchmesser der Stanzöffnung in Querrichtung 4 mm nicht überschreiten. Eine geeignete Abmessung für eine Fassschlaufe an einem medizinischen Absaugschlauch mit kreisförmigen Querschnitt und mit einem äußeren Durchmesser von 5 mm und einem Durchmesser des Lumens von 3 mm ist beispielsweise 5 mm in Längsrichtung und 4 mm in Querrichtung. Eine derartige Fassschlaufe lässt sich gut greifen und ist stabil. Das Einbringen der Stanzöffnung erfolgt nach üblichen Stanzverfahren und kann bei Raumtemperatur oder nach Erhitzen des Schlauches erfolgen.

Gemäß einer zweiten Variante derselben vorgenannten bevorzugten Ausführungsform wird die Fassschlaufe herstellerseitig aus einem mit dem Absaugschlauch unlösbar verbundenem Materialabschnitt geformt. Hierzu formt der Hersteller beispielsweise aus einem Kunststoffmaterial zunächst eine zum Greifen durch ein endoskopisches Instrument angepasste Fassschlaufe. Die Fassschlaufe kann beispielsweise aus einem Kunststoffmaterial geformt werden. Der eine Fassschlaufe umfassende Materialabschnitt wird dann unlösbar mit dem Ende des ersten Abschnitts des Absaugschlauchs verbunden. Eine unlösbare Verbindung kann beispielsweise durch Verpressen, durch Verkleben oder durch Vulkanisieren hergestellt werden. In der Praxis kann der Materialabschnitt neben der Fassschlaufe einen zapfenartigen Anteil umfassen, welcher zur unlösbaren Verbindung mit dem ersten Abschnitt des Absaugschlauchs vorgesehen ist.

Gemäß einer dritten Variante derselben vorgenannten bevorzugten Ausführungsform wird die Fassschlaufe herstellerseitig durch Stanzen einer Öffnung in den Schwammkörper, insbesondere durch Stanzen einer Öffnung in einen Polymerschaumstoff, geformt. Das Stanzen erfolgt vorzugsweise unter Hitzeeinwirkung. Um die Stabilität einer durch Stanzen aus dem Polymerschaumstoff geformten Fassschlaufe zu erhöhen, kann der Polymerschaumstoff nach dem Stanzen im Bereich der Fassschlaufe gehärtet werden. Eine Härtung des die Fassschlaufe umgebenden Materials ist beispielsweise durch Verpressen (insbesondere durch Verpressen unter Hitzeeinwirkung) oder durch chemische Behandlung mit einem aushärtbaren Polymer möglich. Zur Stabilisierung der durch Stanzen aus dem Schaum erzeugten Fassschlaufe können beispielsweise Polymergemische auf der Basis von Silikon, Polyurethan, Polyacetat, Polycarbonat, Polyethylen, Weich- Polyvinychlorid, Polyvinylalkohol oder thermoplastische Polymere verwendet werden. Die Modifikation des die Fassschlaufe umgebenden Materials sollte idealerweise zur Auflösung der Stege des Polymerschaumstoffs führen, so dass ein massives (nicht-porösen) Kunststoffmaterial gebildet wird, welches die Fassschlaufe umläuft. Dies hat den Vorteil, dass sich die Greifzange des endoskopischen Instrumentes beim Loslassen des Saugkörpers dann nicht mehr in den Stegen des Polymerschaumstoffs verfangen kann.

Bei den drei vorgenannten Varianten derselben vorgenannten bevorzugten Ausführungsform weist die Fassschlaufe vorzugsweise eine Länge zwischen 3 mm und 25 mm, besonders bevorzugt zwischen 3 mm und 15 mm, auf. Gemäß einer weiteren besonders bevorzugten Ausführungsform beträgt die Länge zwischen 10 mm und 25 mm. In der Praxis hat sich insbesondere eine Länge der Fassschlaufe von 12 mm (+/-2 mm) als besonders vorteilhaft erwiesen.

Der an dem Absaugschlauch unlösbar angebrachte Schwammkörper umfasst vorzugsweise einen offenzelligen Polymerschaum, insbesondere einen retikulierten Polymerschaumstoff. Bei dem Polymer handelt es sich vorzugsweise um Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan, Kollagen oder um eine Mischung aus den genannten Polymeren.
In einer besonders bevorzugten Ausführungsform umfasst der Schwammkörper einen offenzelligen Polyurethanschaumstoff, der erhältlich ist durch Umsetzung einer Mischung umfassend die Komponenten (i) Polyisocyanat, (ii) Polyol, bevorzugt Polyesterpolyol, (iii) Treibmittel und (iv) Katalysator.
Als Schaumstoffe werden üblicherweise Werkstoffe mit über die gesamte Masse verteilten Zellen (offen, geschlossen oder beides) verstanden. Somit weisen solche Werkstoffe üblicherweise eine Rohdichte (gemäß DIN EN ISO 845) auf, die niedriger ist als die Dichte der Gerüstsubstanz. Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Schaumstoffs wird üblicherweise nach ASTM D 2856-87, Verfahren B) bestimmt. Unter Zellwand wird üblicherweise die die Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke, des übrigen Bereichs der Zellwand auf. Bei dem offenzelligen Schaumstoff kann es sich um einen retikulierten oder um einen nichtretikulierten Schaumstoff handeln. Unter einem retikulierten Schaumstoff wird ein Schaumstoff verstanden, der im Wesentlichen nur Zellstege aufweist. Bei einem retikulierten Schaumstoff sind die Zellwände somit im Wesentlichen entfernt. Die Retikulierung wird üblicherweise in einer Druckkammer, z.B. einer Stahlkammer durchgeführt. Nach Einbringen des Schaumstoffs in die Stahlkammer wird die Luft abgesaugt (bevorzugt zu 50 bis 100 Gew.-%, mehr bevorzugt zu 70 bis 99 Gew.-%) und durch ein Brenngasgemisch, bevorzugt durch ein Gemisch enthaltend Wasserstoff und Sauerstoff, insbesondere im molaren Verhältnis von 2 : 1 ersetzt. Bei der Zündung des Gasgemisches zerreißen die Zellhäute durch die entstehende Hitze- und Druckwelle. Gegebenenfalls erfolgt auch ein zumindest teilweises Aufschmelzen der Zellstege, so dass diese verstärkt werden.

Es hat sich ferner gezeigt, dass vorstehend genannte Aufgaben unerwartet vorteilhaft gelöst werden können, wenn der im Schwammkörper vorhandene Schaumstoff eine spezielle Luftdurchlässigkeit aufweist. In einer bevorzugten Ausführungsform weist der Schaumstoff eine Luftdurchlässigkeit von 1000 bis 8000 l/(m²sec), mehr bevorzugt von 1500 bis 6000 l/(m²sec), noch mehr bevorzugt von 2000 bis 5000 l/(m²sec), besonders bevorzugt von 2300 bis 4000 l/(m²sec) und insbesondere von 2400 bis 3300 l/(m²sec), gemessen gemäß DIN EN ISO 9237 (20 mm Prüfdicke, 20 cm² Prüffläche, 200 Pa Differenzdruck) auf.
Hierbei weist der im Schwammkörper vorhandene Schaumstoff insbesondere eine Bruchdehnung von 100 % bis 400 %, mehr bevorzugt von 120 % bis 300 %, noch mehr bevorzugt von 150 % bis 200 %, gemessen gemäß DIN 53571, auf.
Gemäß einer weiteren bevorzugten Ausführungsform der Erfindung weist der im Schwammkörper vorhandene Schaumstoff eine Zellzahl (= Anzahl der Poren entlang einer Geraden pro laufendem Inch) von mindestens 5 und höchstens 400 Zellen pro Inch auf. Gemäß einer alternativen, gleichfalls sehr vorteilhaften Ausführungsform, beträgt die Zellzahl des im Schwammkörper vorhandenen Schaumstoffs mindestens 10 und höchstens 200 Zellen pro Inch. Die Zellzahl wird bevorzugt mikroskopisch bestimmt.

Der Schwammkörper kann auch zwei oder mehr konzentrisch vorliegende Lagen, insbesondere zwei oder mehr konzentrisch vorliegende Lagen aus Schaumstoff, umfassen. Hierbei ist es besonders vorteilhaft, wenn die äußerste Lage eine Zellzahl zwischen 5 und 200 Zellen pro Inch, vorzugsweise eine Zellzahl zwischen 6 und 100 Zellen pro Inch, besonders bevorzugt eine Zellzahl zwischen 8 und 30 Zellen pro Inch aufweist. Gleichzeitig sollte eine weiter innen vorhandene zweite Lage vorzugsweise zwischen 10 und 400 Zellen pro Inch, besonders bevorzugt zwischen 15 und 200 Zellen pro Inch und insbesondere zwischen 40 und 60 Zellen pro Inch aufweisen. Bei einem derartigen Schwammkörper, welcher zwei oder mehr konzentrisch vorliegende Lagen aus Schaumstoffen mit einer unterschiedlichen Zellzahl umfasst, kann eine besonders effektive Ableitung von Fluiden im Inneren des Schwammkörpers mit einer besonders vorteilhaft ausgestalteten Wundkontaktfläche kombiniert werden.

Gemäß einer weiteren vorteilhaften Ausführungsform ist vorgesehen, dass die Rohdichte des Schaumstoffs zwischen 10 und 350 kg/m³ beträgt, gemessen gemäß DIN EN ISO 845. Besonders bevorzugt beträgt die Rohdichte 15 bis 65 kg/m³, insbesondere 20 bis 45 kg/m³. Die Stauchhärte des für den Schwammkörper vorgesehenen Schaumstoffs beträgt vorzugsweise mindestens 1,5 kPa und höchstens 22,5 kPa, gemessen nach ISO 3386-1.

Der von der Vorrichtung umfasste Schwammkörper kann mit einem Zusatz und/oder Hilfsstoff beschichtet oder imprägniert sein. Bei dem Zusatz oder der Imprägnierung kann es sich beispielsweise um eine die Wundheilung fördernde Salbengrundlage handeln, wobei die Salbengrundlage vorzugsweise zusätzlich einen Quellstoff umfasst. Exemplarisch wird auf die WO2012/167943 verwiesen, welche im Rahmen der Erfindung geeignete Salbengrundlagen beschreibt. Eine im Rahmen der Erfindung besonders geeignete Salbengrundlage, welche zusätzlich einen Quellstoff umfasst, ist in Beispiel 1 der Patentanmeldung WO2012/167943 beschrieben.
Der von der Vorrichtung umfasste Schwammkörper kann alternativ oder zusätzlich zu der vorstehend beschriebenen Salbengrundlage eine antimikrobiell wirksame Substanz umfassen, beispielsweise Polyhexanid. Bei der antimikrobiell wirksamen Substanz kann es sich auch um ein Antibiotikum handeln, beispielsweise um Oxytetracyclin oder um eine Kombination von Bacitracin und Neomycin. Gegebenenfalls kann der Zusatz oder die Imprägnierung zusätzlich zum Antibiotikum oder alternativ eine entzündungshemmende Substanz umfassen, beispielsweise Hydrocortison.
Gemäß einer besonders bevorzugten Ausführungsform umfasst die antimikrobiell wirksame Substanz Silber oder eine Silber-Verbindung.
Der Schwammkörper kann Silber in Form von Silberionen oder in Form von atomarem Silber enthalten. Bevorzugt ist Silber in Form einer Silberbeschichtung auf der Oberfläche des Schwammkörpers aufgebracht. Alternativ kann das Silber innerhalb des Schwammkörpers verteilt sein. Beispielsweise kann bei offenzelligen Schaumstoffen Silber bereits in die härtbare Zusammensetzung mit eingebracht werden. Bevorzugt enthält der im Schwammkörper vorhandene Schaumstoff 0,000001 bis 0,1 Gew.-% Silber, mehr bevorzugt 0,0001 bis 0,01 Gew.-% Silber, bezogen auf das Gesamtgewicht des jeweiligen Schaumstoffs.
Des Weiteren kann der Schwammkörper einen Partikel-förmigen Zusatz umfassen, wobei es sich bei dem Partikel-förmigen Zusatz insbesondere um ein quellfähiges Polymer handelt. Gemäß einer bevorzugten Ausführungsform umfasst Schwammkörper Partikel aus einem superabsorbierenden Polymer (SAP).

Hinsichtlich seiner äußeren Form ist der Schwammkörper im Wesentlichen zylinderförmig ausgebildet, wobei zusätzlich zum stromaufwärts gelegenen Ende hin und/oder zum stromabwärts gelegenen Ende hin abgeschrägt oder gebogen (kuppelförmig) verlaufende Abschnitte vorhanden sein können. Es können auch eine oder mehrere Abschnürungen vorhanden sein, insbesondere eine Abschnürung am stromaufwärts gelegenen Ende des Schwammkörpers und/oder eine Abschnürung am stromabwärts gelegenen Ende des Schwammkörpers. Bei dem Zylinder handelt es sich insbesondere um einen senkrechten Kreiszylinder. Der hier verwendete Begriff "im Wesentlichen zylinderförmig" umfasst auch solche senkrechten Kreiszylinder, welche eine oder zwei abgerundete oder abgeschrägte Kanten aufweisen. Der Schwammkörper weist insbesondere eine Länge von 3 bis 12 cm sowie einen Durchmesser von 1 bis 5 cm auf.

Der Absaugschlauch umfasst bevorzugt einen für medizinische Zwecke geeigneten Kunststoff, insbesondere Silikon, Polyacetat, Polycarbonat, Polyethylen, Polyurethan, Weich-Polyvinychlorid (PVC, welches durch einen Weichmacher modifiziert wurde, beispielsweise durch Tri-2-ethylhexyl Trimellitate - "TOTM"), Polyvinylalkohol, thermoplastische Polymere oder eine Mischung daraus.

Bei der Unterdruckquelle handelt es sich insbesondere eine Saugflasche oder eine elektrisch antreibbare Unterdruckquelle. Eine im Zusammenhang mit der vorliegenden Erfindung besonders geeignete elektrisch antreibbare Unterdruckquelle ist kommerziell unter der Bezeichnung VivanoTec® erhältlich (Paul Hartmann AG, Heidenheim).

Die Erfindung umfasst des Weiteren die Verwendung der Vorrichtung bei der Unterdrucktherapie von endoluminalen Wundstellen im Bereich des Gastrointestinaltraktes.

Die Verwendung der Vorrichtung umfasst die folgenden Schritte, welche die Vorbereitung der Vorrichtung (Anpassung der Länge des Schwammkörpers) und die sich die daran anschließende Unterdrucktherapie betreffen:
i) Bereitstellen einer Vorrichtung, wie in dem vorliegenden Dokument beschrieben, wobei die Vorrichtung eine Unterdruckquelle umfasst.
ii) Endoskopische Vermessung der zu behandelnden Wundstelle bzw. Kavität im Bereich des Gastrointestinaltraktes ("Spiegeln").
iii) Anpassung der Länge des Schwammkörpers an die ermittelte Abmessung der Wundstelle bzw. Kavität, wobei der Schwammkörper an dem den zweiten Abschnitt des Absaugschlauches umschließenden Bereich eingekürzt wird.
iv) Einführen des Saugkörpers bis zur Wundstelle bzw. Kavität.
v) Verbinden des Absaugschlauches mit einer Unterdruckquelle, wobei das Verbinden vorzugsweise mittels eines Konnektors (Schnelltrennverbinder) erfolgt.
vi) Beaufschlagen der Wundstelle bzw. Kavität mit Unterdruck, wobei der Unterdruck über den Absaugschlauch angelegt wird.
Es wird ein Verfahren zur Herstellung einer Vorrichtung zur Verwendung bei der Unterdrucktherapie von endoluminalen Wundstellen im Bereich des Gastrointestinaltraktes beschrieben.

Das Verfahren umfasst die nachfolgenden Schritte, welche allesamt vorzugsweise herstellerseitig durchgeführt werden:
i) Bereitstellen eines für medizinische Zwecke geeigneten Schwammkörpers.
ii) Bereitstellen eines für medizinische Zwecke geeigneten Absaugschlauches, wobei der Absaugschlauch mindestens einen ersten, einen zweiten und einen dritten Abschnitt umfasst.
iii) Auftragen eines Klebstoffes an dem ersten Abschnitt entlang der Längsachse des Absaugschlauches, wobei der erste Abschnitt 10 % bis 90 % der entlang der Achse des Absaugschlauches gemessenen Summe der Längen von erstem und zweitem Abschnitt des Absaugschlauchs umfasst.
iv) Aufbringen des Schwammkörpers auf den Absaugschlauch, wobei der Schwammkörper auf den ersten Abschnitt und auf den zweiten Abschnitt aufgebracht wird, so dass der Schwammkörper den ersten Abschnitt und dem zweiten Abschnitt vollständig umschließt. Nach dem Aushärten des Klebstoffes ergibt sich eine unlösbare Verbindung zwischen dem Schwammkörper und dem ersten Abschnitt des Absaugschlauchs. Der im Bereich des zweiten Abschnitts des Absaugschlauchs vorhandene Anteil des Schwammkörpers kann von dem Anwender (üblicherweise ein Arzt oder eine Ärztin) vor Gebrauch der Vorrichtung bezüglich seiner Länge an die Wundsituation angepasst werden.
Das Verfahren umfasst vorzugsweise ein Sterilisieren und gebrauchsfertiges Konfektionieren der Vorrichtung.
Gemäß einer vorteilhaften Ausführungsform umfasst das Verfahren weiter das Formen einer Fassschlaufe entsprechend der weiter oben näher beschriebenen bevorzugten Ausführungsformen. Die Fassschlaufe kann hierbei gemäß einer ersten Variante der vorteilhaften Ausführungsform des Verfahrens aus einem vierten Abschnitt des Absaugschlauchs geformt werden. Gemäß einer zweiten Variante des Verfahrens kann die Fassschlaufe auch aus einem mit dem Absaugschlauch unlösbar verbundenem Materialabschnitt geformt werden. Die Fassschlaufe kann gemäß einer dritten Variante des Verfahrens aus einem Abschnitt des Schwammkörpers geformt werden.

Gemäß einer besonders bevorzugten Ausführungsform der ersten Variante des Verfahrens wird die Fassschlaufe durch Stanzen einer Öffnung in den Absaugschlauch geformt. Hierzu kann der Absaugschlauch vor dem Stanzvorgang erhitzt werden. Das Einbringen der später bei Gebrauch als Fassschlaufe dienenden Öffnung erfolgt an demjenigen Ende des Absaugschlauches, welches während der Wundbehandlung in die zu behandelnde Wundhöhle eingeführt wird.

Die vorliegende Erfindung betrifft gleichfalls ein Kit zur Verwendung bei der Unterdrucktherapie von endoluminalen Wundstellen im Bereich des Gastrointestinaltraktes. Das Kit umfasst folgende Bestandteile:
- eine Vorrichtung nach einer der vorstehend beschriebenen Varianten und Ausführungsformen, wobei sämtliche Bestandteile der Vorrichtung steril abgepackt vorliegen
- optional ein Gleitgel zum Einführen des Schwammkörpers in ein Lumen des Gastrointestinaltraktes
- optional einen Konnektor zum Verbinden des dritten Abschnitts des Absaugschlauches mit einer Unterdruckquelle, wobei der Konnektor vorzugsweise steril abgepackt vorliegt.

Gemäß einer besonders vorteilhaften Ausführungsform umfasst das Kit mindestens zwei der erfindungsgemäß vorgeschlagenen Saugkörper (unter einem Saugkörper wird im vorliegenden Zusammenhang ein mit einem Absaugschlauch verbundener Schwammkörper verstanden, wobei der Saugkörper die mit der vorliegenden Erfindung vorgeschlagenen Merkmale aufweist), wobei sich die an den mindestens zwei Saugkörpern vorhandenen Schwammkörper bezüglich ihrer Abmessungen unterscheiden. Insbesondere handelt es sich bei den Schwammkörpern um im Wesentlichen zylinderförmig ausgebildete Schwammkörper (senkrechte Kreiszylinder), wobei zusätzlich ein zum wundnahen Ende oder zum Absaugschlauch hin abgeschrägt oder gebogen (kuppelförmig) verlaufender Abschnitt vorhanden sein kann. Der hier verwendete Begriff "im Wesentlichen zylinderförmig" umfasst auch solche senkrechten Kreiszylinder, welche eine oder zwei abgerundete oder abgeschrägte Kanten aufweisen. Jeder der Schwammkörper weist einen Durchmesser d und eine Länge I auf.

Gemäß der hier vorgeschlagenen bevorzugten Ausführungsform des Kits unterscheidet sich jeder der mindestens zwei in dem Kit vorhandenen Schwammkörper in mindestens einer der Größen Durchmesser d und/oder Länge I von jedem anderen der in dem Kit vorhandenen Schwammkörper.

Gemäß einer weiter vorgeschlagenen bevorzugten Ausführungsform der Erfindung unterscheiden sich mindestens zwei der in dem Kit vorhandenen Schwammkörper in nur einer der Größen Durchmesser d oder Länge l von den anderen der in dem Kit vorhandenen Schwammkörper. Da die Länge l erfindungsgemäß durch den Anwender einfach und rasch angepasst werden kann ist es besonders vorteilhaft, wenn sich die in dem Kit vorhandenen Schwammkörper bei identischer Länge l lediglich in dem Durchmesser d unterscheiden. Vorzugsweise umfasst der Kit daher mindestens zwei Schwammkörper, welche eine identische Länge l und unterschiedliche Durchmesser d (d1, d2, bis dn) aufweisen (beispielsweise eine Länge l von jeweils 10 cm und Durchmesser d1 = 1,5 cm und d2 = 3 cm). Besonders bevorzugt unterscheiden sich die Durchmesser (d1, d2, bis dn) der Schwammkörper (bei identischer Länge I) um einen festen Faktor f, welcher insbesondere zwischen 1,3 und 2,5 liegt. Als besonders vorteilhaft zur Versorgung einer Vielzahl von Wundhöhlen hat sich ein Faktor f zwischen 1,5 und 2 erwiesen.
So könnten beispielsweise in einem Kit, welches vier Saugkörper enthält, Schwammkörper mit folgenden Durchmessern d1 bis d4 vorhanden sein:
d1 = 2 cm; d2 = 3 cm; d3 = 4,5 cm; d4 = 6,75 cm
Die Länge l der Schwammkörper beträgt 12 cm. Der Faktor f beträgt bei diesem Beispiel 1,5. Gemäß einer weiteren alternativen Ausführungsform unterscheiden sich die Durchmesser d (d1, d2, bis dn) der Schwammkörper (bei identischer Länge l) um einen fixen Wert, beispielsweise jeweils um 2 cm.
So könnten beispielsweise in einem Kit, welches fünf Saugkörper enthält, Schwammkörper mit folgenden Durchmessern d1 bis d4 vorhanden sein:
d1 = 1 cm; d2 = 2 cm; d3 = 3 cm; d4 = 4 cm; d5 = 5 cm
Die Länge I der Schwammkörper beträgt 10 cm. Der fixe Wert beträgt bei diesem Beispiel 1 cm.

Ein Kit, welches zwei oder drei der erfindungsgemäß vorgeschlagenen Saugkörper enthält, wobei sich die Schwammkörper ausschließlich in ihrem Durchmessern d1 und d2 (zwei Schwammkörper) bzw. d1, d2 und d3 (drei Schwammkörper) voneinander unterscheiden, ist besonders vorteilhaft. Das Kit kann zu vertretbaren Kosten hergestellt werden und deckt alle wesentlichen Wundsituationen ab. Jeder der zwei oder drei Schwammkörper weist dabei herstellerseitig (also vor dem Zuschneiden durch einen Anwender) eine Länge zwischen 5 und 15 cm auf.

Die erfindungsgemäße Vorrichtung kann besonders vorteilhaft zur Unterdruckbehandlung im Gastrointestinaltrakt eingesetzt werden. Sie eignet sich jedoch gleichermaßen zur Verwendung bei der Unterdrucktherapie von ausgedehnten Wundhöhlen außerhalb des Gastrointestinaltraktes.

### Figuren

- Figur 1 a-c: Längenanpassung des Schwammkörpers
- Figur 2 a/b: Erste Variante der erfindungsgemäßen Vorrichtung
- Figur 3 a/b: Zweite Variante der erfindungsgemäßen Vorrichtung
- Figur 4 a/b: Dritte Variante der erfindungsgemäßen Vorrichtung
- Figur 5 a/b: An einem endoskopischen Instrument befestigter Saugkörper
- Figur 6 a/b: Kit, zur Verwendung bei der endoluminalen Unterdrucktherapie
- Figur 7 a - e: Rückbildung einer Abszesshöhle während der Unterdruckbehandlung

### Bezugszeichen

- 1: Absaugschlauch
- 2: Schwammkörper
- 3: Fassschlaufe
- 4: Perforation
- 5: Materialabschnitt
- 6: Zapfen
- 7: Abschnitt des Schwammkörpers, welcher die Fassschlaufe enthält
- 8: Übergangsbereich zwischen Abschnitt 7 und dem Schwammkörper
- 11: Speiseröhre mit seitlich abzweigender Kavität
- 12: Kavität
- 13: Anastomose
- 14: Speiseröhre
- 15: Erster Abschnitt des Absaugschlauchs
- 16: Zweiter Abschnitt des Absaugschlauchs
- 17: Dritter Abschnitt des Absaugschlauchs
- 19: Klebstoffauftrag
- 10, 20, 30, 40, 50, 60: Saugkörper
- 61: Endoskop
- 62: Arbeitskanal
- 63: Mikromechanischer Greifer
- 64: Zange
- 66: Tube mit Gleitmittel
- 100: Medizinisches Kit

### Figur 1

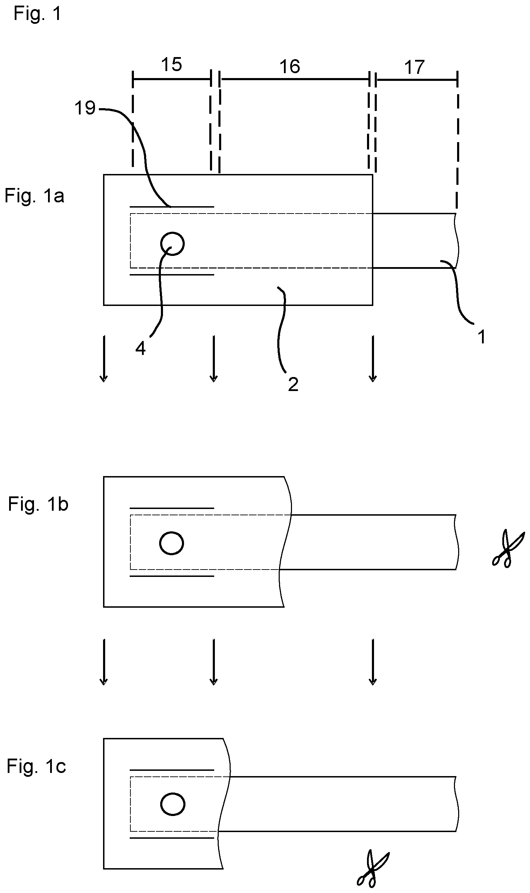

Figur 1 zeigt eine erste Ausführungsform der erfindungsgegenständlichen Vorrichtung schematisch. Die Vorrichtung ist insbesondere zur Verwendung bei der Unterdrucktherapie von endoluminalen Wundstellen im Bereich des Gastrointestinaltraktes vorgesehen, könnte jedoch auch zur Unterdruckbehandlung ausgedehnter Wundhöhlen eingesetzt werden. Die Vorrichtung umfasst einen Schwammkörper (2) und einen mit dem Schwammkörper mittels Klebstoffauftrag (19) unlösbar verbundener Absaugschlauch (1). Ein Schwammkörper (2) und ein mit dem Schwammkörper verbundenen Absaugschlauch (1) wird im vorliegenden Dokument auch als Saugkörper bezeichnet. Der Schwammkörper (2) wird normalerweise aus einem offenzelligen Polymerkunststoff ("Schaumstoff") hergestellt. Der Absaugschlauch weist mindestens drei Abschnitte (15, 16, 17) auf. Herstellerseitig sind der erste Abschnitt (15) und der zweite Abschnitt (16) des Absaugschlauchs vom Schwammkörper umschlossen, während auf den dritten Abschnitt (17) des Absaugschlauchs kein Schaum aufgebracht wird. Der dritte Abschnitt (17) des Absaugschlauchs soll während der Behandlung aus dem Gastrointestinaltrakt heraus nach außen geführt und mit einer Unterdruckquelle verbunden werden. Der Absaugschlauch weist im Bereich des ersten Abschnitts (15) mindestens eine Öffnung (4) auf. In den stromabwärts angeordneten Abschnitten des Absaugschlauchs (1) können weitere Öffnungen (4) vorhanden sein. Im Bereich des ersten Abschnitts (15) ist der Absaugschlauch (1) mit einem Klebstoffauftrag (19) versehen, welcher eine unlösbare Verbindung zwischen Absaugschlauch Schwammkörper bewirkt. Im Bereich des zweiten Abschnitts (15) des Absaugschlauchs ist herstellerseitig kein Klebstoffauftrag vorgesehen. Entsprechend kann der Schwammkörper, wie in Figuren 1 b/c schematisch dargestellt, von seinem stromabwärts gelegenen Ende her schrittweise verkürzt werden. Diese Längenanpassung wird erst von dem Anwender (normalerweise ein/eine Arzt/Ärztin) kurz vor der Verwendung der Vorrichtung durchgeführt. Die Längenanpassung des Saugkörpers wird anhand einer vor dem Eingriff durchgeführten endoskopischen Vermessung der Wundstelle vorgenommen. Bei dem in Figur 1b gezeigten Saugkörper wurde ein Ring-Segment des Schaumstoff-Körpers (2) vom Anwender mittels Schere oder Skalpell entfernt. Der zur Wundstelle hin orientierte stromaufwärts vorhandene Anteil des Schwammkörpers wird bei der in Figur 1b gezeigten Variante der Erfindung durch die Längenanpassung nicht modifiziert. Dies setzt voraus, dass wie in Figur 1 dargestellt, der erste Abschnitt (15) des Absaugschlauchs stromaufwärts des zweiten Abschnitts des Absaugschlauchs (16) angeordnet ist. Eine umgekehrte Anordnung (nicht dargestellt) von erstem und zweitem Abschnitt wäre denkbar, wobei dann eine Längenanpassung nur an dem stromaufwärts vorhandenen zweiten Abschnitt (17) vorgenommen werden kann. Figur 1c zeigt beispielhaft eine durch den Anwender vorgenommene Längenanpassung, bei der der um den zweiten Abschnitt (16) des Absaugschlauchs (1) vorhandene Schaum (2) vollständig abgeschnitten wurde.

### Figuren 2 a/b

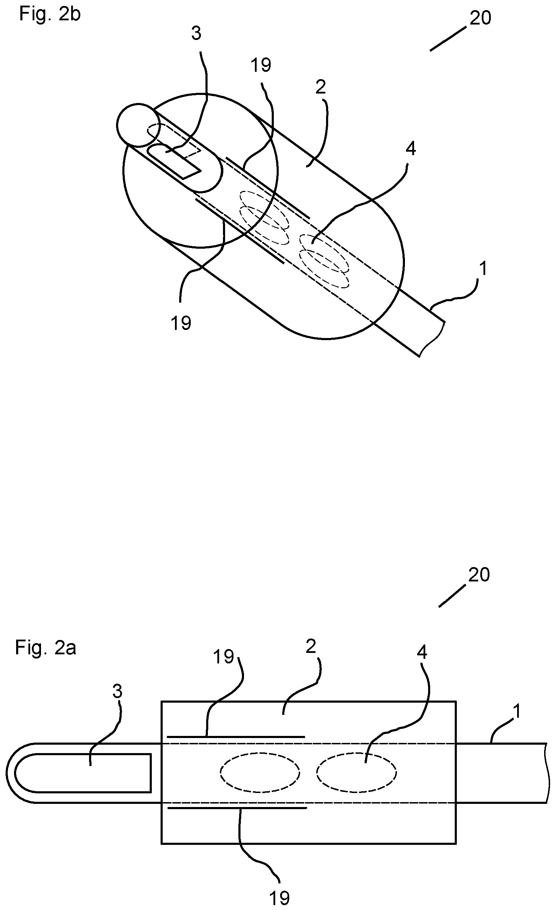

Bei der in den Figuren 2 a/b schematisch gezeigten Ausführungsform der Saugkörpers (20) ist zusätzlich eine Fassschlaufe (3) vorhanden, welche das Ergreifen des Saugkörpers (20) durch ein endoskopisches Instrument erleichtert. Die Fassschlaufe wird herstellerseitig durch einen Stanzvorgang direkt aus dem Absaugschlauch (1) hergestellt. In demselben Stanzvorgang, in dem die Fassschlaufe (3) geformt wird, können gleichzeitig weitere Öffnungen (4) in den Absaugschlauch (1) eingebracht werden. Diese zusätzlichen Öffnungen (4) werden an dem von dem Schwammkörper (2) umschlossen Abschnitt des Absaugschlauches (1) eingebracht und sind zur Applikation des Unterdrucks und zum Ansaugen von Fluiden aus dem Wundraum erforderlich. Die Öffnungen (4) können, wie in Figur 2 b angedeutet, in dem Schlauch gegenüberliegend vorhanden sein. Sie können alternativ entlang der Schlauchachse zueinander versetzt vorliegen (nicht dargestellt).

Die Fassschlaufe (3) ragt bei dem dargestellten Beispiel aus dem Schwammkörper (2) heraus. Die Fassschlaufe ist entsprechend an einem vierten Abschnitt des Absaugschlauchs vorhanden, welcher ebenso wie der dritte Abschnitt (17) nicht vom Schwammkörper umschlossen wird. Da die Fassschlaufe (3) bei dem in den Figuren 2 a/b gezeigten Beispiel mit dem Lumen des Absaugschlauches (1) in einem Kontinuum steht, entsteht während der Behandlung auch ein Sog über die Fassschlaufe (3). Dieser Effekt hat sich als vorteilhaft erwiesen, da die Ableitung zäher oder partikelhaltiger Sekrete hierdurch verbessert wird. Grundsätzlich wäre es jedoch auch möglich, das Lumen des Absaugschlauches (1) gegenüber der Fassschlaufe (3) zu isolieren, so dass der Unterdruck ausschließlich über den Schwammkörper mit dem Wundraum kommunizieren kann (in Figur 2 a/b nicht dargestellt). Bei dem in den Figuren 2 a/b gezeigten Beispiel wurde die Fassschlaufe (3) in einen Schlauch gestanzt, dessen Ende einen abgerundeten und geschlossenen Endabschnitt aufweist. Obgleich es grundsätzlich möglich wäre, die Fassschlaufe (3) in einen Schlauch einzubringen, dessen Endabschnitt offen vorliegt und eine glatte Schnittfläche aufweist (in Figuren 2 a/b nicht dargestellt), hat sich ein abgerundeter und geschlossener Abschluss des Absaugschlauches als vorteilhaft erwiesen, da hierdurch eine besonders schonende Einbringung des Saugkörpers (20) in den Wundraum erleichtert wird.

Lediglich beispielhaft wird im Folgenden eine mögliche Dimensionierung für einen Schwammkörper (2), einen Absaugschlauch (1) und die aus dem Absaugschlauch (1) geformte Fassschlaufe (3) angegeben:

| | |
|---|---|
| Außendurchmesser des Absaugschlauchs: | 4,6 mm |
| Wandstärke des Absaugschlauchs: | 1,0 mm |
| Durchmesser des Lumens des Saugschlauches: | 2,6 mm |
| Länge der Fassschlaufe: | 12 mm |
| Seitliche Ausdehnung der Fassschlaufe: | 2,0 mm |

Der in Figuren 2 a/b beispielhaft dargestellte Absaugschlauch (1) ist aus Weich-PVC.

Der Schwammkörper (2) kann beispielsweise einen hydrophoben, retikulierten Polyester-Polyurethan-Schaumstoff umfassen, mit einer Rohdichte gemäß ISO 845 von 26,0 kg/m³, mit einer Stauchhärte gemäß DIN EN ISO 3386-1 von 3,4 kPa, mit einer Bruchdehnung gemäß DIN 53571 von 290 % und mit einer Zellzahl (mikroskopisch bestimmt an einer an der Oberfläche des Schaumes angelegten geraden Linie) von 26 Zellen pro Inch.

Alternativ kann der Schwammkörper (2) einen hydrophoben, retikulierten Polyester-Polyurethan-Schaumstoff umfassen, dessen Rohdichte gemäß ISO 845 28,0 kg/m³, dessen Stauchhärte gemäß DIN EN ISO 3386-1 3,6 kPa, dessen Bruchdehnung gemäß DIN 53571 335 %, und dessen Zellzahl (mikroskopisch bestimmt an einer an der Oberfläche des Schaumes angelegten geraden Linie) 38 Zellen pro Inch beträgt.

Gemäß einer weiteren Ausführungsform kann der Schwammkörper (2) einen hydrophoben Polyether-Polyurethan-Schaumstoff mit einer Rohdichte gemäß ISO 845 von 23,0 kg/m³, mit einer Stauchhärte gemäß DIN EN ISO 3386-1 von 3,8 kPa, mit einer Bruchdehnung gemäß DIN 53571 von 310 % und mit einer Zellzahl (mikroskopisch bestimmt an einer an der Oberfläche des Schaumes angelegten geraden Linie) von 31 Zellen pro Inch umfassen.

Es wäre auch denkbar, dass der Schwammkörper (2) zwei oder mehr konzentrisch angeordnete Lagen aus einem oder mehreren der vorgenannten Schaumstoffe umfasst.

### Figuren 3 a/b

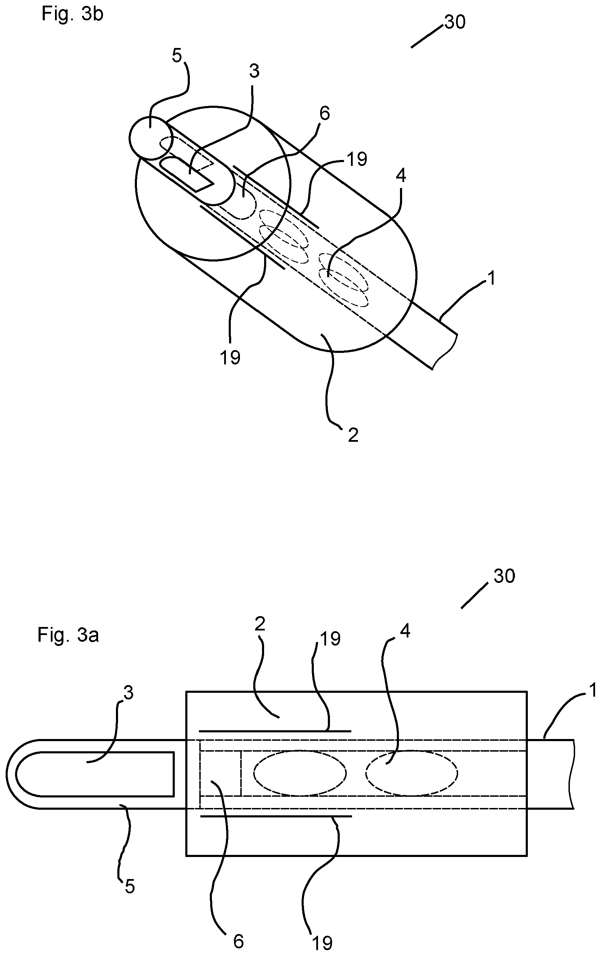

Die Figuren 3 a/b zeigen in schematisierter Darstellung eine weitere Ausführungsform der Erfindung, bei welcher wie bei der in Figur 2 gezeigten Ausführungsform eine Fassschlaufe (3) vorhanden ist. Die Fassschlaufe (3) ist aus einem mit dem ersten Abschnitt (15) des Absaugschlauchs (1) unlösbar verbundenen Materialabschnitt (5) geformt. Der Materialabschnitt (5) ist an seinem zum Saugkörper (30) hin zeigenden Ende zapfenartig mit dem Absaugschlauch (1) verbunden. Hierzu wird der zapfenförmige Anteil (6) des Materialabschnittes (5) herstellerseitig in den ersten Abschnitt (15) des Absaugschlauchs (1) eingeschoben und mit diesem verklebt. Der Außendurchmesser des Materialabschnittes (5) sollte vorzugsweise dem Außendurchmesser des Absaugschlauches (1) entsprechen, so dass ein glatter Übergang vom Absaugschlauch (1) zum Materialabschnitt (5) vorhanden ist, wie in Figuren 3 a/b dargestellt. An seinem vom Saugkörper (30) weg zeigenden Ende ist an dem Materialabschnitt (5) eine Fassschlaufe (3) vorhanden. Der Materialabschnitt (5) umfasst vorzugsweise ein Kunststoffmaterial. Im Inneren des Materialabschnittes (5) ist vorzugsweise ein zentrales Lumen (in Figuren 3 a/b nicht dargestellt) vorhanden, welches mit dem Lumen des Absaugschlauches (1) kommunizieren kann. Hierdurch wird der im Absaugschlauch (1) vorhandene Unterdruck bis zur Fassschlaufe (3) weitergeleitet. Dies hat sich als vorteilhaft erwiesen, da hierdurch eine besonders effektive Ableitung von Wundsekreten gewährleistet werden kann. Das vom Absaugschlauch (1) weg zeigende Ende des Materialabschnittes (5) ist vorzugsweise abgerundet ausgeführt, da hierdurch eine schonende Applikation des Saugkörpers (30) in den Wundbereich erleichtert wird.

### Figur 4 a/b

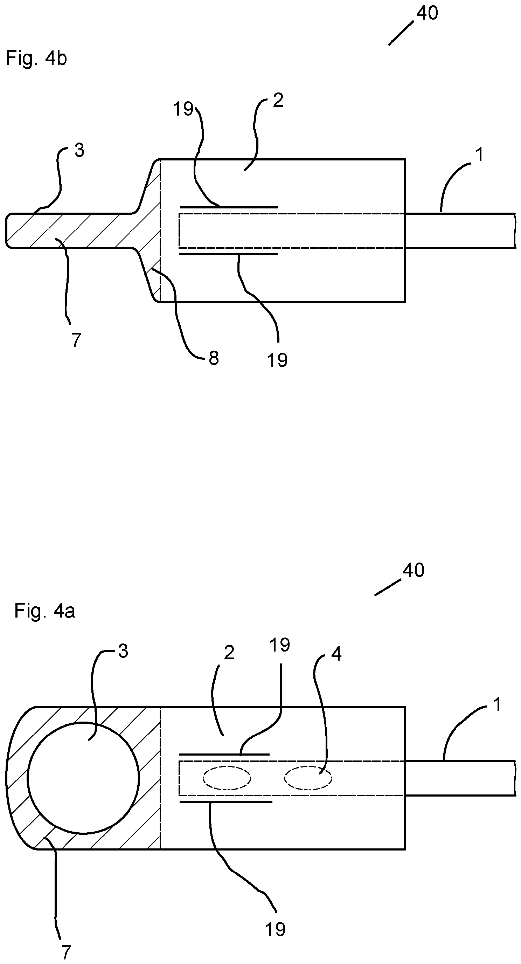

Bei der in den Figuren 4 a/b schematisch gezeigten weiteren Ausführungsform der Erfindung ist gleichfalls eine herstellerseitig bereitgestellte Fassschlaufe (3) vorgesehen. Die Fassschlaufe (3) wird herstellerseitig aus einem Abschnitt des Schwammkörpers (2) geformt. Vorzugsweise umfasst der Schwammkörper (2) einen Polymerkunststoff oder besteht aus einem Polymerkunststoff. Figur 4 a zeigt die Fassschlaufe (3) in der Aufsicht, während sie in Figur 4 b von der Seite gezeigt ist (bei der Darstellung gemäß Figur 4 b wurde der Saugkörper (40) also gegenüber Figur 4 a um 90° entlang der Schlauchachse gedreht). Die Fassschlaufe (3) wurde aus dem zylinderförmigen Schwammkörper (2) durch zwei aufeinander folgende Stanzvorgänge geformt. Bei dem ersten Stanzvorgang werden aus dem Kreiszylinder zwei Abschnitte mit kreissegmentförmigen Querschnitt gestanzt. Vorzugsweise sollte ein abgeschrägter Übergangsbereich (8) zwischen dem planaren Abschnitt (7) und dem zylinderförmiger Schwammkörper (2) vorhanden sein, wie in Figur 4b angedeutet. Vor dem zweiten Stanzvorgang wird der Saugkörper um 90° entlang der Schlauchachse gedreht. Dann wird in den plattenförmig ausgebildeten Bereich (7) eine Öffnung gestanzt, um die Fassschlaufe (3) auszubilden. Die Öffnung kann beispielsweise kreisförmig, wie in Figur 4b dargestellt, ausgebildet sein. Vorzugsweise wird der die Fassschlaufe (3) umfassende Bereich (7) nach dem Stanzen gehärtet, um die Stabilität der Fassschlaufe (3) zu erhöhen. Die Härtung kann beispielsweise durch einen unter Hitzeeinwirkung durchgeführten Pressvorgang oder durch chemische Behandlung des Schaumstoffes erfolgen.

### Figuren 5 a/b

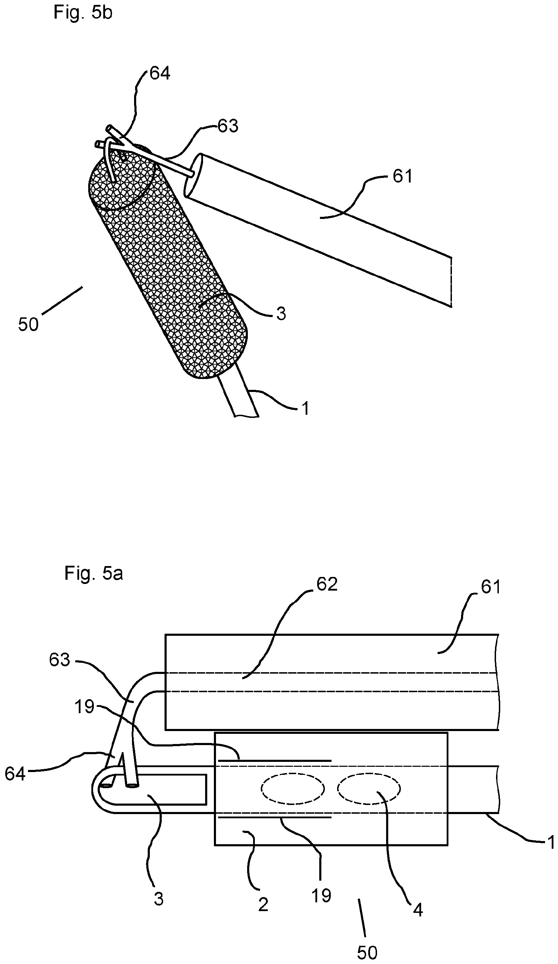

Die schematischen Darstellungen in Figur 5 a/b zeigen einen an einem endoskopischen Instrument (61) lösbar befestigten Saugkörper (50), wobei der Saugkörper eine Fassschlaufe (3) aufweist. Wie aus Figur 5a erkennbar, verläuft durch das endoskopische Instrument (61) ein Arbeitskanal (62), welcher zum Einführen und Bedienen von mikromechanischen Geräten vorgesehen ist. Bei dem mikromechanischen Gerät handelt es sich im Zusammenhang mit der vorliegenden Erfindung um einen Greifer mit einer abwinkelbaren Spitze. Am Ende des Greifers (63) ist eine bewegliche endoskopische Mikro-Zange (64) vorhanden. Die Mikro-Zange (64), welche in den Figuren 5 a/b nur angedeutet ist, kann mechanisch oder elektronisch ferngesteuert werden. Des Weiteren umfasst das endoskopische Instrument (61) eine Kamera (in Figur 5 a/b nicht dargestellt) zur Spiegelung des zu behandelnden Lumen. Der endoskopische Greifer (63) mit endoskopischer Zange (64) kann den Saugkörper (50) an der Fassschlaufe (3) ergreifen. Die Verbindung von endoskopischem Instrument (61) und Saugkörper (50) mittels endoskopischer Zange (64) und Fassschlaufe (3) stellt der behandelnde Arzt noch außerhalb des Patienten her. Die Längsachse des Saugkörpers (50) kann dann parallel zur Längsachse des Endoskops (61) angeordnet werden. Beim Einführen des Endoskops (61) in das Lumen des Gastrointestinaltraktes und gegebenenfalls in einen von dem Lumen abzweigenden Abszess wird der Saugkörper "huckepack" mitgezogen. Am Zielort kann der Arzt die an dem Greifer (63) vorhandene endoskopische Zange (64) öffnen und den Saugkörper freisetzen. Anschließend wird das Endoskop (61) zurückgezogen. Nach der Entfernung des Endoskops (61) kann an den Absaugschlauch (1) Unterdruck angelegt werden.

### Figuren 6 a/b

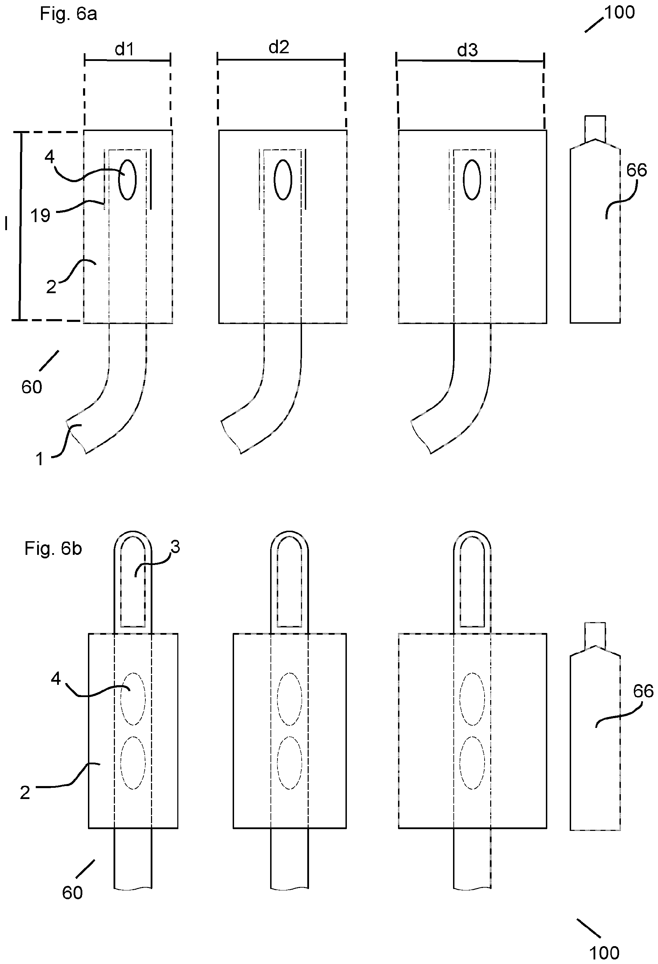

Die Figuren 6 a/b zeigen ein medizinisches Kit bzw. Verbandset (65), welches zur Verwendung bei der Unterdrucktherapie des Gastrointestinaltraktes vorgesehen ist. Bei den in den Figuren 6 a/b dargestellten Beispielen enthält das Kit jeweils drei Saugkörper (60). Weiterhin enthält das Kit jeweils eine Tube (66) mit Gleitgel. Das Gleitgel kann vor dem Einbringen des Saugkörpers (60) in den Gastrointestinaltrakt auf den Schwammkörper aufgetragen werden, um die Applikation des Saugkörpers zu erleichtern. Vorzugsweise wird das Gleitgel erst aufgebracht, nachdem eine Längenanpassung des Saugkörpers durch den Anwender erfolgt ist. In Figur 6 a ist ein Kit gezeigt, welches Saugkörper enthält, die der in Figur 1 gezeigten Ausführungsform entsprechen. Figur 6 b zeigt eine Ausführungsform der in dem Kit enthaltenen Saugkörper entsprechend dem in Figur 2 gezeigten Beispiel (die Saugkörper weisen eine Fassschlaufe (3) auf). Bei den in Figuren 6 a/b beispielhaft dargestellten medizinischen Kits weisen alle drei der in dem Kit enthaltenen Schwammkörper jeweils eine identische Länge auf, während sich die Durchmesser (d1, d2, d3) voneinander unterscheiden. Mit einer derartigen Kombination von Saugkörpern können alle typischen, bei der Unterdrucktherapie des Gastrointestinaltraktes auftretenden Wundsituationen, insbesondere Kavitäten, behandelt werden. Hierzu wählt der Anwender nach der endoskopischen Vermessung des Wundbereichs einen Saugkörper mit einen für die Wundsituation geeigneten Durchmesser d aus. Danach kann der Anwender auf einfache und unkomplizierte Weise den am ausgewählten Saugkörper vorhandenen Schwammkörper in seiner Länge I an die Wundkavität anpassen, indem er den Schwammkörper an seinem stromabwärts vorhandenen Ende zuschneidet.

Die Länge I der Schwammkörper kann bei den in den Figuren 6 a/b gezeigten Kits beispielsweise jeweils 10 cm betragen. Geeignete Abmessungen für den Durchmesser sind beispielsweise: d1 = 1 cm, d2 = 2 cm und d3 = 4 cm.

Vorzugsweise werden herstellerseitig Kits zur Verfügung gestellt, welche entweder zur Verwendung zur Behandlung des oberen oder alternativ zur Behandlung des unteren Gastrointestinaltraktes vorgesehen sind.

### Figuren 7 a - e

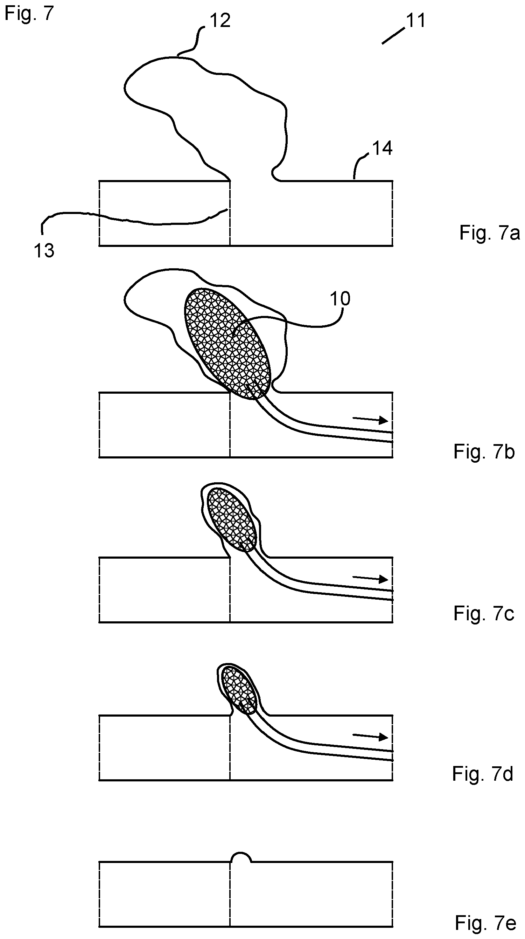

Figuren 7 a - e zeigen einen durch Unterdruckbehandlung unterstützten Heilungsverlauf bei einer aus einer Speiseröhre (14) abzweigenden Kavität (12) in schematischer Darstellung. Die in Figur 7 gezeigte Unterdruckbehandlung einer endoluminalen Kavität, welche an sich aus dem Stand der Technik bekannt ist, kann besonders vorteilhaft mit einer erfindungsgemäßen Vorrichtung durchgeführt werden. Die in den Figuren 7 a - e dargestellten Fluidsammelkörper sind lediglich schematisch gezeichnet. Die erfindungsgemäßen Merkmale sind in den Figuren 7 a - e nicht dargestellt. Eine aus der Speiseröhre seitlich abzweigende Kavität (12), wie in Figur 7 a dargestellt, kann unter anderem infolge einer chirurgisch erzeugten Anastomose (13) entstehen (beispielsweise nach einer Tumorexzision). Vor der Unterdruckbehandlung wird die Kavität (12) endoskopisch gespiegelt (nicht dargestellt), um ihre Abmessung zu ermitteln. Anhand der endoskopischen Vermessung erfolgt eine Längenanpassung des Schwammkörpers, wobei der im Bereich des zweiten Abschnittes des Absaugschlauchs vorhandene Schwamm eingekürzt wird. Nachdem ein derart an die Abmessungen der Kavität angepasster Fluidsammelkörper (10) in die Wundhöhle (12) eingebracht ist, kann über den Absaugschlauch (1) Unterdruck angelegt werden (Figur 7 b). Die Unterdruckapplikation bewirkt eine Ableitung von Wundsekreten und unterstützt die Kontraktion der Kavität (12), wie in Figuren 7 b - d angedeutet. Nach einer permanenten Unterdruckbehandlung von einigen Tagen bis zu mehreren Wochen ist die Kavität bereits so weit geschrumpft, dass der Fluidsammelkörper aus den Wundbereich entfernt werden kann (Figur 7 e).

## Patentansprüche

1. Vorrichtung zur Verwendung bei der Unterdrucktherapie von endoluminalen Wundstellen im Bereich des Gastrointestinaltraktes,
umfassend
einen Schwammkörper (2) und einen mit dem Schwammkörper (2) mittels Klebstoffauftrag (19) unlösbar verbundenen Absaugschlauch (1),
wobei der Absaugschlauch (1) mindestens einen ersten (15), einen zweiten (16) und einen dritten Abschnitt (17) aufweist,
und wobei der Schwammkörper (2) herstellerseitig den ersten (15) und den zweiten Abschnitt (16) des Absaugschlauches (1) umschließt,
und wobei der Schwammkörper (2) den dritten Abschnitt (17) des Absaugschlauchs (1) nicht umschließt,
und wobei des Weiteren im Bereich des ersten Abschnitts (15) des Absaugschlauchs (1) mindestens eine Öffnung (4) vorhanden ist,
**dadurch gekennzeichnet,**
**dass** der Absaugschlauch (1) im Bereich des ersten Abschnitts (15) herstellerseitig mittels Klebstoffauftrag (19) mit dem Schwammkörper (2) unlösbar verbunden ist, und dass der Absaugschlauch (1) im Bereich des zweiten Abschnitts (16) keinen Klebstoffauftrag (19) aufweist,
wobei der erste Abschnitt (15) des Absaugschlauchs (1) im Fluidsystem stromaufwärts des zweiten Abschnitts (16) vorhanden ist.

2. Vorrichtung nach Anspruch 1, wobei der erste Abschnitt (15) 10 % bis 90 % der entlang der Achse des Absaugschlauches (1) gemessenen Summe der Längen von erstem (15) und zweitem Abschnitt (16) des Absaugschlauchs (1) umfasst.

3. Vorrichtung nach Anspruch 1 oder 2, wobei der Absaugschlauch (1) einen vierten Abschnitt umfasst, welcher stromaufwärts des ersten Abschnitts (15) vorhanden ist und welcher von dem Schwammkörper (2) nicht umschlossen ist, so dass der vierte Abschnitt aus dem Schwammkörper (2) herausragt.

4. Vorrichtung nach einem oder mehreren der vorangehenden Ansprüche, wobei der Schwammkörper (2) einen offenzelligen Schaum, insbesondere einen offenzelligen Polyurethanschaum, umfasst.

5. Vorrichtung nach einem oder mehreren der vorangehenden Ansprüche, wobei der Schwammkörper (2) im Wesentlich zylinderförmig ausgebildet ist und eine Länge von 3 bis 10 cm sowie einen Durchmesser von 0,5 bis 5 cm aufweist.

6. Vorrichtung nach einem oder mehreren der vorangehenden Ansprüche, wobei der Absaugschlauch (1) Silikon, Polyacetat, Polycarbonat, Polyethylen, Polyurethan, Weich-Polyvinychlorid, Polyvinylalkohol, thermoplastische Polymere oder eine Mischung daraus umfasst.

7. Vorrichtung nach einem oder mehreren der vorangehenden Ansprüche, wobei an der Vorrichtung eine bereits herstellerseitig angebrachte Fassschlaufe (3) vorhanden ist, und wobei die Fassschlaufe (3) aus einem Abschnitt des Schwammkörpers (2) geformt ist, aus einem Abschnitt des Absaugschlauchs (1) geformt ist oder aus einem mit dem Absaugschlauch (1) unlösbar verbundenem Materialabschnitt geformt ist.

8. Vorrichtung nach einem oder mehreren der vorangehenden Ansprüche, wobei der Absaugschlauch (1) einen Konnektor zum Verbinden des dritten Abschnitts des Absaugschlauches (1) mit einer Unterdruckquelle umfasst.

9. Vorrichtung nach einem oder mehreren der vorangehenden Ansprüche, weiterhin umfassend eine Unterdruckquelle, insbesondere eine Saugflasche oder eine elektrisch antreibbare Unterdruckquelle.

10. Kit zur Verwendung bei der Unterdrucktherapie von endoluminalen Wundstellen im Bereich des Gastrointestinaltraktes, umfassend
- eine Vorrichtung nach einem oder mehreren der Ansprüche 1 bis 8, wobei sämtliche Bestandteile der Vorrichtung steril abgepackt vorliegen
- optional ein Gleitgel zum Einführen des Schwammkörpers in ein Lumen des Gastrointestinaltraktes.

11. Kit nach Anspruch 10, wobei das Kit mindestens zwei der Vorrichtungen nach einem oder mehreren der Ansprüche 1 bis 8 umfasst,
und wobei jeder der Schwammkörper (2) einen Durchmesser d und eine Länge I aufweist,
**dadurch gekennzeichnet, dass** sich jeder der in dem Kit vorhandenen Schwammkörper (2) in mindestens einer der Größen ausgewählt aus Durchmesser d und/oder Länge I von jedem anderen der in dem Kit vorhandenen Schwammkörper (2) unterscheidet.

12. Kit nach Anspruch 11, wobei sich mindestens zwei der Schwammkörper (2) in nur einer einzigen Größe ausgewählt aus Durchmesser d oder Länge I von jedem der anderen in dem Kit vorhandenen Schwammkörper (2) unterscheiden.

13. Kit nach Anspruch 12, wobei der Kit mindestens zwei Schwammkörper (2) umfasst, welche eine identische Länge I und einen unterschiedlichen Durchmesser d aufweisen.

14. Kit nach Anspruch 13, wobei das Kit genau zwei oder drei der Vorrichtungen enthält, und wobei die Länge der Schwammkörper (2) 5 cm bis 15 cm beträgt.

## Claims

1. Device for use in negative pressure therapy of endoluminal wound sites in the gastrointestinal tract, comprising
a sponge body (2), and a suction hose (1) connected non-detachably to the sponge body (2) by means of adhesive (19),
wherein the suction hose (1) has at least a first portion (15), a second portion (16) and a third portion (17),
and wherein the sponge body (2), at the production stage, encloses the first portion (15) and the second portion (16) of the suction hose (1),
and wherein the sponge body (2) does not enclose the third portion (17) of the suction hose (1),
and wherein, furthermore, at least one opening (4) is present in the region of the first portion (15) of the suction hose (1),
**characterized in that**
the suction hose (1) is connected non-detachably to the sponge body (2) by means of adhesive (19) at the production stage in the region of the first portion (15), and **in that** the suction hose (1) has no adhesive (19) in the region of the second portion (16) ,
wherein the first portion (15) of the suction hose (1) is present in the fluid system upstream from the second portion (16).

2. Device according to Claim 1, wherein the first portion (15) comprises 10% to 90% of the sum of the lengths of the first portion (15) and second portion (16) of the suction hose (1), measured along the axis of the suction hose (1).

3. Device according to Claim 1 or 2, wherein the suction hose (1) comprises a fourth portion, which is present upstream from the first portion (15) and which is not enclosed by the sponge body (2), such that the fourth portion protrudes from the sponge body (2).

4. Device according to one or more of the preceding claims, wherein the sponge body (2) comprises an open-cell foam, in particular an open-cell polyurethane foam.

5. Device according to one or more of the preceding claims, wherein the sponge body (2) is substantially cylindrical and has a length of 3 to 10 cm and a diameter of 0.5 to 5 cm.

6. Device according to one of more of the preceding claims, wherein the suction hose (1) comprises silicone, polyacetate, polycarbonate, polyethylene, polyurethane, soft polyvinyl chloride, polyvinyl alcohol, thermoplastic polymers or a mixture thereof.

7. Device according to one or more of the preceding claims, wherein a gripping loop (3), already fitted at the production stage, is present on the device, and wherein the gripping loop (3) is formed from a portion of the sponge body (2), is formed from a portion of the suction hose (1) or is formed from a material portion connected non-detachably to the suction hose (1).

8. Device according to one or more of the preceding claims, wherein the suction hose (1) comprises a connector for connecting the third portion of the suction hose (1) to a negative pressure source.

9. Device according to one or more of the preceding claims, further comprising a negative pressure source, in particular a suction bottle or an electrically driven negative pressure source.

10. Kit for use in negative pressure therapy of endoluminal wound sites in the gastrointestinal tract, comprising
- a device according to one or more of Claims 1 to 8, wherein all the constituent parts of the device are present in sterile packaging,
- optionally, a lubricant gel for inserting the sponge body into a lumen of the gastrointestinal tract.

11. Kit according to Claim 10, wherein the kit comprises at least two of the devices according to one or more of Claims 1 to 8,
and wherein each of the sponge bodies (2) has a diameter d and a length l,
**characterized in that** each of the sponge bodies (2) present in the kit differs, in at least one of the sizes chosen from diameter d and/or length l, from every other sponge body (2) present in the kit.

12. Kit according to Claim 11, wherein at least two of the sponge bodies (2) differ, in only a single size chosen from diameter d or length l, from each of the other sponge bodies (2) present in the kit.

13. Kit according to Claim 12, wherein the kit comprises at least two sponge bodies (2) which have an identical length 1 and a different diameter d.

14. Kit according to Claim 13, wherein the kit contains precisely two or three of the devices, and wherein the length of the sponge bodies (2) measures 5 cm to 15 cm.

## Revendications

1. Dispositif à utiliser lors de la thérapie par pression négative des plaies endoluminales dans la zone du tractus gastrointestinal,
comprenant
un corps spongieux (2) et un tuyau d'aspiration (1) relié de manière indissociable au corps spongieux (2) au moyen d'une application d'adhésif (19),
le tuyau d'aspiration (1) possédant au moins une première (15), une deuxième (16) et une troisième portion (17), et le corps spongieux (2), par la fabrication, entourant la première (15) et la deuxième portion (16) du tuyau d'aspiration (1),
et le corps spongieux (2) n'entourant pas la troisième portion (17) du tuyau d'aspiration (1),
et au moins une ouverture (4) étant en outre présente dans la zone de la première portion (15) du tuyau d'aspiration (1),
**caractérisé en ce**
**que** le tuyau d'aspiration (1), par la fabrication, est relié de manière indissociable au corps spongieux (2) dans la zone de la première portion (15) au moyen d'une application d'adhésif (19),
et **en ce que** le tuyau d'aspiration (1) ne présente pas d'application d'adhésif (19) dans la zone de la deuxième portion (16),
la première portion (15) du tuyau d'aspiration (1) se trouvant en amont de la deuxième portion (16) dans le système de fluide.

2. Dispositif selon la revendication 1, la première portion (15) comprenant 10 % à 90 % de la somme des longueurs de la première (15) et de la deuxième portion (16) du tuyau d'aspiration (1) mesurées le long de l'axe du tuyau d'aspiration (1).

3. Dispositif selon la revendication 1 ou 2, le tuyau d'aspiration (1) comprenant une quatrième portion qui se trouve en amont de la première portion (15) et qui n'est pas entourée par le corps spongieux (2), de sorte que la quatrième portion fait saillie hors du corps spongieux (2) .

4. Dispositif selon une ou plusieurs des revendications précédentes, le corps spongieux (2) comprenant une mousse à alvéoles ouvertes, notamment une mousse de polyuréthane à alvéoles ouvertes.

5. Dispositif selon une ou plusieurs des revendications précédentes, le corps spongieux (2) étant de configuration sensiblement cylindrique et présentant une longueur de 3 à 10 cm ainsi qu'un diamètre de 0,5 à 5 cm.

6. Dispositif selon une ou plusieurs des revendications précédentes, le tuyau d'aspiration (1) comprenant du silicone, du polyacétate, du polycarbonate, du polyéthylène, du polyuréthane, du polychlorure de vinyle, de l'alcool polyvinylique, des polymères thermoplastiques ou un mélange de ceux-ci.

7. Dispositif selon une ou plusieurs des revendications précédentes, une boucle de préhension (3) déjà montée lors de la fabrication étant présente sur le dispositif, et la boucle de préhension (3) étant formée à partir d'une portion du corps spongieux (2), étant formée à partir d'une portion du tuyau d'aspiration (1) ou étant formée à partir d'une portion de matériau reliée de manière indissociable au tuyau d'aspiration (1).

8. Dispositif selon une ou plusieurs des revendications précédentes, le tuyau d'aspiration (1) comprenant un connecteur destiné à relier la troisième portion du tuyau d'aspiration (1) à une source de pression négative.

9. Dispositif selon une ou plusieurs des revendications précédentes, comprenant en outre une source de pression négative, notamment un flacon d'aspiration ou une source de pression négative pouvant être entraînée électriquement.

10. Kit destiné à être utilisé lors de la thérapie par pression négative des plaies endoluminales dans la zone du tractus gastrointestinal, comprenant
- un dispositif selon une ou plusieurs des revendications 1 à 8, tous les éléments constitutifs du dispositif se présentant sous emballage stérile
- en option, un gel lubrifiant servant à l'insertion du corps spongieux dans un lumen du tractus gastrointestinal.

11. Kit selon la revendication 10, le kit comprenant au moins deux des dispositifs selon une ou plusieurs des revendications 1 à 8,
et chacun des corps spongieux (2) présentant un diamètre d et une longueur l,
**caractérisé en ce que** chaque corps spongieux (2) présent dans le kit se différencie de chaque autre corps spongieux (2) présent dans le kit par au moins l'une des grandeurs choisies parmi le diamètre d et/ou la longueur l.

12. Kit selon la revendication 11, au moins deux des corps spongieux (2) ne se différenciant de chaque autre corps spongieux (2) présent dans le kit que par une seule des grandeurs choisies parmi le diamètre d ou la longueur l.

13. Kit selon la revendication 12, le kit comprenant au moins deux corps spongieux (2), lesquels présentent une longueur l identique et un diamètre d différent.

14. Kit selon la revendication 13, le kit comprenant exactement deux ou trois dispositifs, et la longueur des corps spongieux (2) étant de 5 cm à 15 cm.
